# EUROPEAN PATENT APPLICATION

(11) **EP 1 405 920 A2**
(43) Date of publication of application: **07.04.2004**
(21) Application number: 03022028.9
(22) Date of filing: 01.10.2003
(51) Int. Cl.: C12Q 1/68

(54) **Improved FRET process**

(30) Priority: 02.10.2002 EP 02022228
(71) Applicant: Roche Diagnostics GmbH, 68305 Mannheim (DE); F.HOFFMANN-LA ROCHE AG, 4070 Basel (CH)
(72) Inventor: Sagner, Gregor, Dr., 82377 Penzberg (DE); Heindl, Dieter, Dr., 82396 Paehl (DE); Bechler, Ingrid, 82538 Geretsried (DE); Krause, Christina, 82377 Penzberg (DE)

(57) **Abstract**

The present invention is directed to an improved pair of FRET hybridization probes hybridizing adjacently to a target nucleic acid sequence, one member of said pair of FRET hybridization probes comprising (i) a nucleotide sequence entity which is substantially complementary to the sequence of the target nucleic acid, (ii) a fluorescent entity, said entity being either the FRET donor entity or the FRET acceptor entity, (iii) a spacer entity connecting said nucleotide sequence entity and said fluorescent entity, characterized in that the length of said spacer entity is a chain of at least 15 atoms.

## Description

The invention originates from the field of Real Time PCR. More specifically, the invention is directed to an improved design of Hybridization Probes.

### Prior art Background

In kinetic real-time PCR, the formation of PCR products is monitored in each cycle of the PCR. The amplification is usually measured in thermocyclers which have additional devices for measuring fluorescence signals during the amplification reaction. A typical example of this is the Roche Diagnostics LightCycler (Cat. No. 2 0110468). The amplification products are for example detected by means of fluorescent labeled hybridization probes which only emit fluorescence signals when they are bound to the target nucleic acid or in certain cases also by means of fluorescent dyes that bind to double-stranded DNA. A defined signal threshold is determined for all reactions to be analyzed and the number of cycles Cp required to reach this threshold value is determined for the target nucleic acid as well as for the reference nucleic acids such as the standard or housekeeping gene. The absolute or relative copy numbers of the target molecule can be determined on the basis of the Cp values obtained for the target nucleic acid and the reference nucleic acid (Roche Diagnostics LightCycler operator manual(Cat. No. 2 0110468))

There exist different formats for the detection amplified DNA:

### a) DNA binding dye formate

Since the amount of double stranded amplification product usually exceeds the amount of nucleic acid originally present in the sample to be analyzed, double-stranded DNA specific dyes may be used, which upon excitation with an appropriate wavelength show enhanced fluorescence only if they are bound to double-stranded DNA. Preferably, only those dyes may be used which, like SYBR Green I for example, do not affect the efficiency of the PCR reaction.

All other formats known in the art require the design of a fluorescent labeled hybridization probe which only emits fluorescence upon binding to its target nucleic acid.

### b) TaqMan probes

A single-stranded hybridization probe is labeled with two components. When the first component is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the Taq Polymerase during the subsequent elongation phase. As a result the excited fluorescent component and the quencher are spatially separated from one another and thus a fluorescence emission of the first component can be measured (US5,538,848).

### c) Molecular Beacons

These hybridization probes are also labeled with a first component and with a quencher, the labels preferably being located at both ends of the probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

### d) FRET hybridization probes

The FRET hybridization probe test formate is especially useful for all kinds of homogenous hybridization assays (Matthews, J. A. and Kricka, L. J., Anal Biochem 169 (1988) 1-25.. It is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected.

When annealed to the target sequence, the hybridization probes must be located very close to each other, in a head to tail arrangement. Usually, the gap between the labeled 3' end of the first probe and the labeled 5' end or the second probe is as small as possible, i.e.1-5 bases. This allows for a close vicinity of the FRET donor compound and the FRET acceptor compound, which is typically 10-100 Angstroem.

Alternatively to monitoring the increase in fluorescence of the FRET acceptor component, it is also possible to monitor fluorescence decrease of the FRET donor component as a quantitative measurement of hybridization event.

In particular, the FRET hybridization probe formate may be used in real time PCR, in order to detect the amplified target DNA. Among all detection formats known in the art of real time PCR, the FRET-hybridization probe format has been proven to be highly sensitive, exact and reliable (WO 97/46707; WO 97/46712; WO 97/46714). Yet, the design of appropriate FRET hybridization probe sequences may sometimes be limited by the special characteristics of the target nucleic acid sequence to be detected.

As an alternative to the usage of two FRET hybridization probes, it is also possible to use a fluorescent-labeled primer and only one labeled oligonucleotide probe (Bernard, P. S., et al., Anal Biochem 255 (1998) 101-7). In this regard, it may be chosen arbitrarily, whether the primer is labeled with the FRET donor or the FRET acceptor compound.

Besides PCR and real time PCR, FRET hybridization probes are used for melting curve analysis. In such an assay, the target nucleic acid is amplified first in a typical PCR reaction with suitable amplification primers The hybridization probes' may already be present during the amplification reaction or added subsequently. After completion of the PCR-reaction, the temperature of the sample is constitutively increased, and fluorescence is detected as long as the hybridization probe was bound to the target DNA. At melting temperature, the hybridization probes are released from their target, and the fluorescent signal is decreasing immediately down to the background level. This decrease is monitored with an appropriate fluorescence versus temperature-time plot such that a first derivative value can be determined, at which the maximum of fluorescence decrease is observed.

However, for kinetic real time PCR as well as for melting curve analysis, tremendous differences in absolute signal intensities have been observed for different hybridization probes, although being labeled with the same couple of fluorescent dyes. Moreover, this phenomenon is independent from the couple of fluorescent dyes which is used.

The reason for the observed effect is unknown, although one may speculate that it could be due to quenching or dequenching effects of G residues which have been disclosed previously in various systems (WO 01/36668, Seidel, C. A. M., et al., J Phys Chem 100 (1996) 5541-53).

In most cases, G residues causing quenching effects are usually located in close spatial vicinity to the respective fluorescent compound (EP 1 046 717). Moreover, based on this effect, it is possible in some cases to set up an assay, wherein fluorescent emission of an unhybridized labeled probe is quenched by internal residues and hybridization can monitored due to a dequenching effect occurring as soon as the probe is being hybridized to a complementary target sequence (WO 01/73118).

In other cases, however, the reason for comparatively low signal intensities that are observed for hybridization probes which are labeled with specific couples of FRET dyes is far from being understood. Yet, this observed effect is highly disadvantageous especially with respect to the design of multiplex assays, characterized in that within one reaction vessel, one or more target sequences are amplified and quantitatively analyzed with two or multiple hybridization probes or pairs of FRET hybridization probes. Thus there is a need in the art for an improved design of FRET Hybridization probes wherein the absolute fluorescent signal is enhanced.

### Brief description of the invention

This problem is resolved by the design of a pair of hybridization probes according to the present invention.

It is directed to a pair of hybridization probes, wherein one member of said pair of hybridization probes comprises
- a nucleotide sequence entity which is substantially complementary to the sequence of the target nucleic acid
- a fluorescent entity, and
- a spacer entity connecting said nucleotide sequence entity and said fluorescent entity in such a way that upon hybridization of said pair of probes to the target DNA, the distance of the two fluorescent compounds which generate the FRET pair is increased as compared to a pair of FRET hybridization probes with no spacer entities between the nucleotide sequence entity and the fluorescent entity.

In a first aspect, the present invention is directed to a pair of FRET hybridization probes hybridizing adjacently to a target nucleic acid sequence, a first member of said pair of hybridization probes comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity, said spacer entity comprising a chain of at least 15 atoms, wherein 2 atoms of said connecting chain of at least 15 atoms are carrying negatively charged substituents.

In one specific embodiment, the spacer entity is composed of a chain of nucleotide residues, characterized in that the length of said chain is 1-10, preferably 2-7 and most preferred 3-5 A, T. or C nucleotide residues. When the probe is bound to its target DNA, the nucleotides representing the spacer are not capable of hybridizing to the target DNA.

It has been proven to be advantageous, if the spacer according to the invention is present within the member of the pair of FRET hybridization probes carrying the FRET donor entity.

In a second aspect, the present invention is directed to a pair of FRET hybridization probes hybridizing adjacently to a target nucleic acid sequence, the first member of said pair of FRET hybridization probes comprising
- a nucleotide sequence entity which is substantially complementary to the sequence of the target nucleic acid
- a fluorescent entity, said entity being the FRET donor entity
- a spacer entity connecting said nucleotide sequence entity and said fluorescent entity,
the second member of said pair of FRET hybridization probes comprising
- a nucleotide sequence entity which is substantially complementary to the sequence of the target nucleic acid
- a fluorescent entity, said entity being the FRET acceptor entity
- a spacer entity connecting said nucleotide sequence entity and said fluorescent entity, said spacer entity being different from the spacer entity of said first member of said pair of FRET hybridization probes,
wherein the length of said spacer entity of said first member and the length of said spacer entity from said second member of said pair of FRET hybridization probes differ in size at least by chain of 15 atoms.

If the spacer entity is composed of nucleotide residues, this aspect of the invention may be defined as a pair of FRET hybridization probes hybridizing adjacently to a target nucleic acid sequence, the first member of said pair of FRET hybridization probes comprising
- a nucleotide sequence entity which is substantially complementary to the sequence of the target nucleic acid
- a fluorescent entity, said entity being the FRET donor entity
- a spacer entity connecting said nucleotide sequence entity and said fluorescent entity, said spacer entity comprising a number of n1=0-15 nucleotide residues which are not capable of hybridizing to the target DNA
the second member of said pair of FRET hybridization probes comprising
- a nucleotide sequence entity which is substantially complementary to the sequence of the target nucleic acid
- a fluorescent entity, said entity being the FRET acceptor entity
- a spacer entity connecting said nucleotide sequence entity and said fluorescent entity, said spacer entity comprising a number of n2=0-15 nucleotide residues which are not capable not hybridizing to the target DNA,
wherein the value of n1 differs from the value of n2 by by a natural number between 1 and 10, preferably by a natural number between 2-7 and most preferably by a natural number between 3-5.

In a third aspect, the present invention is directed to a set of at least 3 oligonucleotides, characterized in that a first oligonucleotide and a second oligonucleotide are capable of acting as a pair of amplification primers for a template dependent nucleic acid amplification reaction, further characterized in that said first oligonucleotide and said third oligonucleotide are each labeled with one corresponding member of a FRET pair consisting of a FRET donor entity and a FRET acceptor entity, characterized in that
either said first oligonucleotide or said third oligonucleotide are comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity,
wherein said spacer entity comprises a connecting chain of at least 15 atoms.

In another aspect, the invention is also directed to a composition comprising a nucleic acid sample and a pair of hybridization disclosed above.

Also, the present invention provides a kit comprising a pair of hybridization probes or a set of oligonucleotides as disclosed above. Preferably, the kit may contain at least one other component selected from a group consisting of nucleic acid amplification primers, template dependent nucleic acid polymerase, deoxynucleoside triphosphates and a buffer for template dependent nucleic acid amplification reaction.

The inventive pair of hybridization probes may be used for any homogenous or heterogenous method for qualitative or quantitative detection of a nucleic acid sequence in a biological sample. In particular, the FRET hybridization probes according to the invention may be used for a method, wherein a part of said nucleic acid present in said sample, which comprises a target nucleic acid sequence substantially complementary to the sequences of said pair of FRET hybridization probes, is amplified by a nucleic acid amplification reaction, in particular by a polymerase chain reaction. In a specific embodiment, fluorescence emission of either the FRET donor entity or emission of the FRET acceptor entity is monitored in real time.

In a further aspect, the present invention provides an improved method for the determination of the melting profile of a hybrid consisting of a target nucleic acid and a pair of FRET hybridization probes disclosed above, characterized in that the fluorescence emission is determined as a function of temperature.

In a still further aspect, the invention is directed to a method for chemical solid phase synthesis of multiple oligonucleotides comprising
a) Preparation of a dye labeled CPG- (N)n, characterized in that N are arbitrarily chosen nucleotide residues different from G and n=1-10
b) Solid phase synthesis of a first oligonucleotide having a first sequence using the CPG prepared in step a)
c) Solid phase synthesis of at least a second oligonucleotide having a second sequence using the CPG prepared in step a)

### Brief description of the figures

### Figure 1

Fluorescence versus cycle number plot of the real time PCR experiment disclosed in example 2 using Fluorescein/JA286 FRET hybridization probes to detect a FactorV amplicon having one spacer according to the invention.
1) -: FRET hybridization probes without any spacer
2) 5A: FRET donor probe carrying a 5 A residue spacer
3) 3A: FRET donor probe carrying a 3 A residue spacer
4) 5T: FRET acceptor probe carrying a 5 T residue spacer
5) 3T: FRET acceptor probe carrying a 3T residue spacer

### Figure 2

1^{st} derivative of fluorescence versus temperature plot showing the melting curve analysis of the experiment disclosed in example 3 using Fluorescein/JA286 FRET hybridization probes comprising one spacer according to the invention.
1) -: FRET hybridization probes without any spacer
2) 5A: FRET donor probe carrying a 5 A residue spacer
3) 3A: FRET donor probe carrying a 3 A residue spacer
4) 5T: FRET acceptor probe carrying a 5 T residue spacer
5) 3T: FRET acceptor probe carrying a 3T residue spacer

### Figure 3

Fluorescence versus cycle number plot of the real time PCR experiment disclosed in example 4 using different pairs of FRET hybridization probes to detect a G6PDH amplicon. The experiment compares FRET pairs with Fluorescein as a FRET donor being linked to the oligonucleotide either directly ("neg") or by 5A residue spacer ("5A") according to the invention.

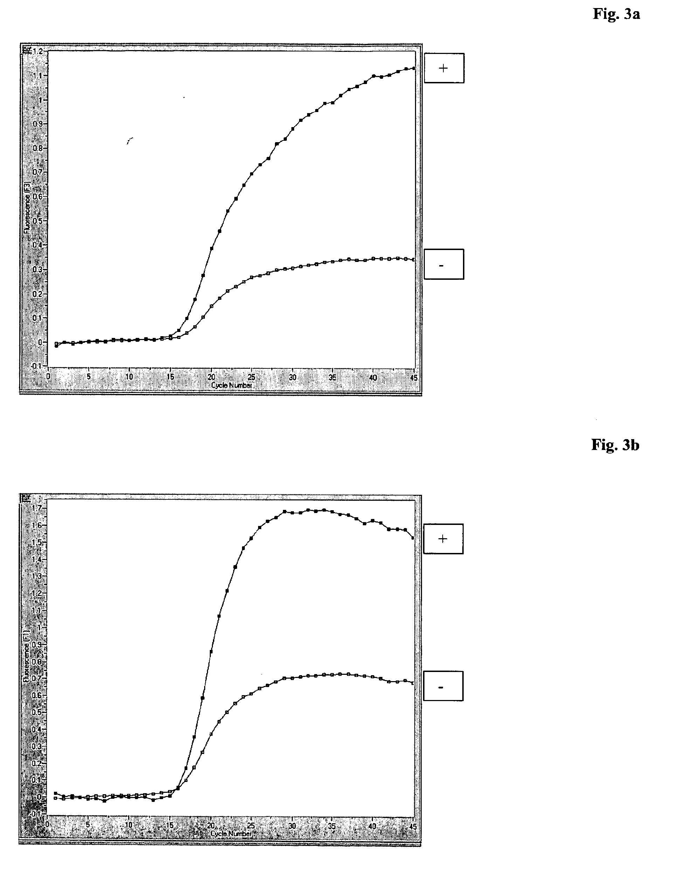

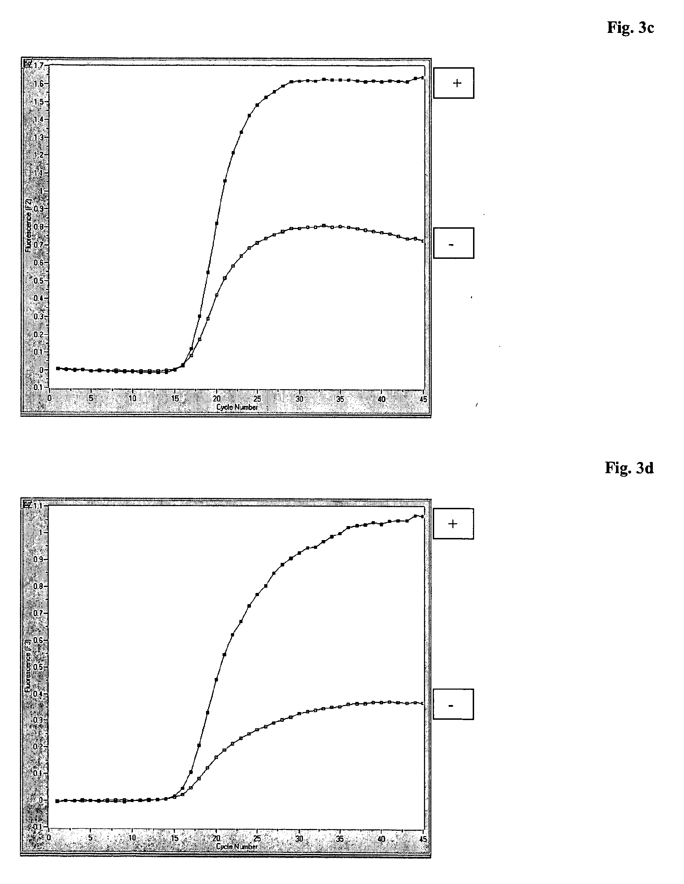

Fig. 3a: -/+ Fluorescein/JA286 compared to Fluorescein-5A/JA286
Fig. 3b: -/+ Fluorescein/LCRed640 Fluorescein-5A/LCRed640
Fig. 3c: -/+ Fluorescein/Cy5 compared to Fluorescein-5A/Cy5
Fig. 3d: -/+ Fluorescein/LCRed 705 compared to Fluorescein-5A/LCRed 705

### Figure 4

Fluorescence versus cycle number plot of the real time PCR experiment disclosed in example 5 using Fluorescein/JA286 FRET hybridization probes or Fluorescein/LCRed705 hybridization probes to detect a FactorV amplicon having with different spacers according to the invention.

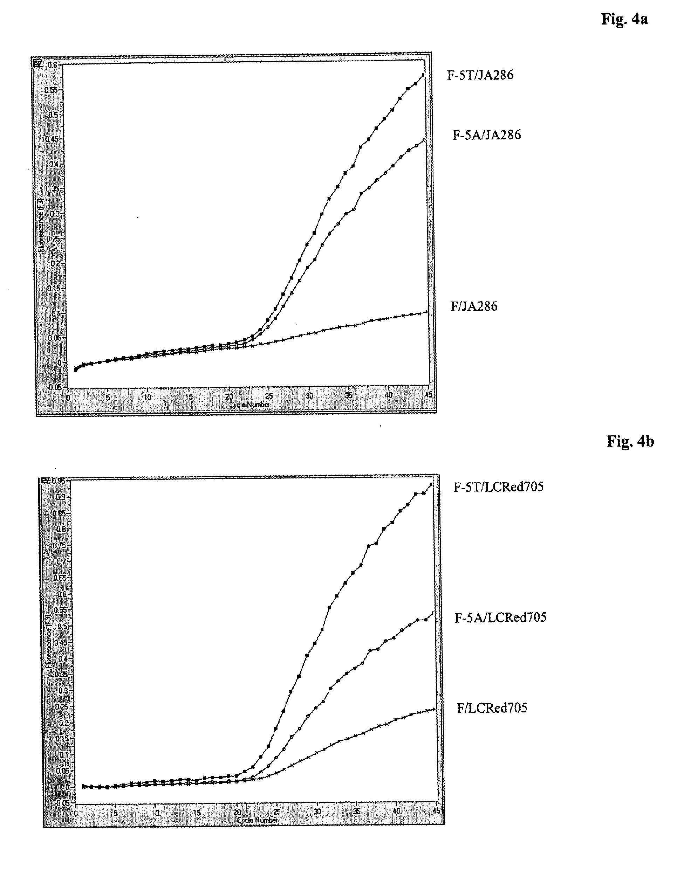

Fig. 4a: Fluorescein/JA286 FRET hybridization probes

### F/JA286:

FRET pair Fluorescein/JA286 without spacer

### F-5A/JA286:

FRET pair Fluorescein/JA286, the Fluorescein donor probe carrying a 5A spacer

### F-5T/JA286:

FRET pair Fluorescein/JA286, the Fluorescein donor probe carrying a 5T spacer

### Fig. 4b: Fluorescein/LCRed705 hybridization probes

F/LCRed705:

FRET pair Fluorescein/LCRed705 without spacer

F5A/LCRed705:

FRET pair Fluorescein/LCRed705, the Fluorescein donor probe carrying a 5A spacer

F5T/LCRed705:

FRET pair Fluorescein/LCRed705, the Fluorescein donor probe carrying a 5T spacer

### Detailed description of the invention

The present invention is based on the surprising observation that in all situations investigated by the inventors so far, a limited spatial separation of the FRET donor compound and the FRET acceptor compound surprisingly results in an increased fluorescent resonance energy transfer process.

As it is known in the art, the FRET hybridization probe test format is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected.

In this context, the present invention is directed to a pair of FRET hybridization probes, wherein the fluorescent label of one member of said pair of hybridization probes is connected with the oligonucleotide chain by a specific spacer moiety . As a consequence, upon hybridization of both probes to the target DNA, the distance of the two fluorescent compounds which generate the FRET pair is increased as compared to conventional FRET hybridization probes. Surprisingly however, the inventors have observed that in all cases investigated so far, this results in an increased fluorescent signaling.

According to the invention, the desired effect of increasing the fluorescence resonance energy transfer and thus fluorescent signaling is achieved by a pair of FRET hybridization probes hybridizing adjacently to a target nucleic acid sequence, wherein one member of said pair of hybridization probes comprises
- a nucleotide sequence entity which is substantially complementary to the sequence of the target nucleic acid
- a fluorescent entity, and
- a spacer entity connecting said nucleotide sequence entity and said fluorescent entity in such a way that upon hybridization of said pair of probes to the target DNA, the distance of the two fluorescent compounds which generate the FRET pair is increased as compared to a pair of FRET hybridization probes with no spacer entities between the nucleotide sequence entity and the fluorescent entity.

In the context of the present invention, the term "substantially complementary" shall mean, that the respective sequences specifically hybridize to each other under standard annealing conditions. The length of the nucleotide sequence entity which is substantially complementary to the sequence of the target nucleic acid may vary between 10 and 40 nucleotide residues. Preferably, but depending on the AT content of the target nucleic acid, said length is between 15 and 30 nucleotide residues. At least, a perfect Watson Crick base pairing between more than 85 % of the residues constituting the hybrid is required. In addition, a perfect complementarity over a segment of 10 constitutive nucleotide residues is required.

In the context of the present invention, the term "hybridizing adjacently" shall mean that in case the two hybridization probes are hybridized to the target nucleic acid, there exists either no or only a small gap ranging over 0-10 and preferably 1-2 complementary nucleotide residues between the two probes with respect to the target nucleic acid sequence

In a first aspect, the new invention provides a pair of FRET hybridization probes hybridizing adjacently to a target nucleic acid sequence, a first member of said pair of hybridization probes comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity, said spacer entity comprises a connecting chain of at least 15 atoms. said spacer entity comprising a connecting chain of at least 15 atoms,
wherein 2 atoms of said connecting chain of at least 15 atoms are carrying negatively charged substituents.

For means of clarification regarding this first aspect of the invention, it is emphasized that in order to obtain the desired improved FRET signaling, only one member of said pair of FRET hybridization probe carries a spacer entity comprising a chain of at least 15 atoms, whereas the other member is directly labeled with a fluorescent compound by any conventional method known in the art.

For further clarification, it is emphasized that in the context of the present invention, the term "spacer entity" comprises all items between the fluorescent chromophore which are not participating in mesomeric effects and on the other side all items which are not part of the ribose entity of the either 5' or 3' terminal nucleotide residue.

As explained above, the spacer entity present in one member of an inventive pair of hybridization probes comprises a connecting chain of at least 15 atoms. Yet, in order to obtain the desired effect of improved FRET signaling the chain should not exceed more than a hundred atoms. Preferably, the chain of connecting atoms is 20 - 60 and most preferably 30 - 45 atoms. This results in a distance of about 20 - 150 Angstroems, preferably 25 - 90 Angstroems and most preferably 40 - 70 Angstroems between the nucleotide sequence entity and the fluorescent entity connected by the spacer.

It needs to be noted that as a consequence the distance between the donor entity and the FRET acceptor entity, when the FRET probes are bound to the target sequence, is also increased. Yet, usage of FRET hybridization probes nevertheless surprisingly results in an increased fluorescence signaling. The increase in the distance corresponds to the Angstroems valuables given above for the length of the spacer entities according to the present invention.

In order to obtain the desired effect of an improved FRET process it is essential that the spacer entity is at least partially negatively charged. This effect is obtained if at least two atoms of said connecting chain of at least 15 atoms are carrying negatively charged substituents. Preferably, this effect is obtained by means of introducing at least two phosphate groups into the connecting chain representing the spacer. Alternatively, the spacer entity may contain at least two side chains, having free hydroxy or acidic groups such as a carboxy group.

In a preferred embodiment, the spacer entity is composed of a chain of nucleotide residues, which are not capable of hybridizing to the target DNA. In this regard, the two fluorescent moieties are still brought in a sufficiently reasonable vicinity to each other, if the numbers of nucleotide residues is between 1 and 10, preferably 2 and 7, and most preferably 3-5.

Thus, one oligonucleotide of an inventive pair of FRET hybridization probes is designed in such a way that 3' or 5' to the nucleotide sequence entity which is substantially complementary to the sequence of the target nucleic acid, there are 1-10, preferably 2-7, and most preferably 3-5 additional nucleotide residues which act as a spacer entity. The 3' or 5' terminal end of the terminal residue not hybridizing to the target DNA, is labeled with one FRET entity according to standard protocols known in the art.

Preferably, said additional non-hybridizing residues are A,T or C residues. Highly preferred, more than 70 % of said additional residues are A, C, or T residues. Most preferred, said additional residues are a string of homo-A, homo-T, or homo-C.

In another preferred embodiment which is not mutually exclusive to the previous one, it is the FRET donor entity which is connected to the sequence entity according to the invention by a spacer entity comprising a connecting chain of at least 15 atoms. Moreover, it has been proven to be of particular advantage, if for the inventive FRET hybridization probes, Fluorescein is used as a FRET donor entity.

In a second aspect, the new invention provides a pair of FRET hybridization probes hybridizing adjacently to a target nucleic acid sequence, the first member of said pair of FRET hybridization probes comprising
- a nucleotide sequence entity which is substantially complementary to the sequence of the target nucleic acid
- a fluorescent entity, said being the FRET donor entity
- a spacer entity connecting said nucleotide sequence entity and said fluorescent entity,
the second member of said pair of FRET hybridization probes comprising
- a nucleotide sequence entity which is substantially complementary to the sequence of the target nucleic acid
- a fluorescent entity, said entity being the FRET acceptor entity
- a spacer entity connecting said nucleotide sequence entity and said fluorescent entity, said spacer entity being different from the spacer entity of said first member of said pair of FRET hybridization probes,
wherein the length of said spacer entity of said first member and the length of said spacer entity of said second member of said pair of FRET hybridization probes differ in size at least by chain of 15 atoms.

Preferably, at least 2 of these 15 different atoms carry negatively charged substituents. Most preferably, these at least 2 atoms are represented by phosphate groups. Moreover, the preferred spacer characteristics with respect to its size as disclosed for the first aspect of the invention also apply for the difference between said two spacer entities.

In case a chain of nucleotide residues shall be used as an appropriate spacer entity, the invention is also directed to a pair of FRET hybridization probes hybridizing adjacently to a target nucleic acid sequence, the first member of said pair of FRET hybridization probes comprising
- a nucleotide sequence entity which is substantially complementary to the sequence of the target nucleic acid
- a fluorescent entity, said entity being the FRET donor entity
- a spacer entity connecting said nucleotide sequence entity and said fluorescent entity, said spacer entity comprising a number of n1=0-15 nucleotide residues which are not capable of hybridizing to the target DNA
the second member of said pair of FRET hybridization probes comprising
- a nucleotide sequence entity which is substantially complementary to the sequence of the target nucleic acid
- a fluorescent entity, said entity being the FRET acceptor entity
- a spacer entity connecting said nucleotide sequence entity and said fluorescent entity, said spacer entity comprising a number of n2=0-15 nucleotide residues which are not capable not hybridizing to the target DNA,
wherein the value of n1 differs from the value of n2 by by a natural number between 1 and 10, preferably by a natural number between 2-7 and most preferably by a natural number between 3-5.

It is also within the scope of this specific embodiment, if said spacer entities of the two members of said pair of FRET hybridization probes are capable of forming non-covalent interactions such as nucleotide base pairing interactions with each other. In other words, both FRET hybridization probes are carrying a different number of additional nucleotide residues not hybridizing to the target nucleic acid such that a part of the additional residues of the first hybridization probe may form base pairing interactions with the nucleotide residues of the second hybridization probe thus forming a partial stem structure, when hybridized to the target nucleic acid.

Preferably, these base pairing interactions are A/T base pairing interactions. In addition, it is also within the scope of the present invention, if the stem structure formed by the nucleotides generating the base pairing interactions optionally comprises single mismatches.

In addition, the present invention is also applicable to the primer/probe format useful for the detection of PCR amplification products. For this format, one primer is internally labeled with one fluorescent compound that participates in the FRET process.

Therefore, in the context of all different aspects of the present invention, the term "pair of FRET hybridization probes" is also being defined as comprising a pair of oligonucleotides, characterized in that one of said oligonucleotides together with a third oligonucleotide is capable of acting as a primer for a polymerase amplification reaction (PCR). In this case, a FRET signal occurs, when during or subsequently to an amplification reaction, the second oligonucleotide carrying a fluorescent component is being hybridized to the amplicon generated by means of PCR.

As a consequence, the present invention also provides a set of at least 3 oligonucleotides, characterized in that a first oligonucleotide and a second oligonucleotide are capable of acting as a pair of amplification primers for a template dependent nucleic acid amplification reaction, further characterized in that said first oligonucleotide and said third oligonucleotide are each labeled with one corresponding member of a FRET pair consisting of a FRET donor entity and a FRET acceptor entity, such that either said first oligonucleotide or said third oligonucleotide comprise
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity,
- wherein said spacer entity comprises a connecting chain of at least 15 atoms, preferably consists of a chain of 1-10 and most preferably consist of 3-5 nucleotide residues.

Preferably, it is the third oligonucleotide which comprises the spacer entity according t o the invention, since the usage of a labeled primer requires an internal labeling different from a conventional 5' or 3' labeling which may be performed in automated processes known in the art. Most preferably, said third oligonucleotide is labeled with the FRET donor compound.

Also preferably, at least 2 atoms of said connecting chain of at least 15 atoms carry negatively charged substituents. Most preferably, these at least 2 atoms are represented by phosphate groups. Moreover, the preferred spacer characteristics with respect to its size as disclosed for the first aspect of the invention also apply.

When using the inventive pairs of FRET hybridization probes or the inventive set of oligonucleotides disclosed above, the intensity of fluorescence emission from the FRET acceptor entity of a pair of FRET hybridization probes according to the invention, when hybridized to its target sequence is detectably increased compared to the intensity of fluorescence emission of a FRET acceptor entity of a comparative pair of FRET hybridization probes hybridized to the same target DNA, if said comparative pair of FRET hybridization probes is identical to said pair of hybridization probes with the exception that no member of said comparative pair of hybridization probes does comprise a spacer entity connecting said nucleotide sequence entity and said fluorescent entity.

In this context, the term "detectably increased" shall mean that a difference between the two mentioned pairs of FRET hybridization probes can be monitored in a conventional real time PCR assay, for example a LightCycler instrument (Roche Applied Sciences). Preferably, however, the detectable difference is more than 20%.

The degree of fluorescence signal increase depends significantly on the target nucleic acid sequence and on the pair of FRET dyes used. In certain cases the fluorescence signal increase can exceed more than 200 %. Fluorescence signal increase is not only observed in conventional hybridization assays. As will be shown in the examples, it is also detectable in real time PCR quantification and in melting curve analysis.

In another aspect, the invention is directed to a composition comprising a nucleic acid sample and a pair of hybridization probes according to claims 1-6. Prior to analysis, such a composition is mixed with a sample to be analyzed thus forming a new composition additionally containing the target nucleic acid to become detected.

In a further aspect, the present invention is also directed to various methods and applications of using the inventive oligonucleotide pairs and compositions disclosed above.

For example, the pair of FRET hybridization probes according to the invention may be used for qualitative or quantitative detection of a nucleic acid sequence in a biological sample, wherein the nucleic acid present in said sample is hybridized with said pair of FRET hybridization probes according to the invention.

In one embodiment, such a method may be a typical hybridization assay. The hybridization may take place either in solution, or alternatively, either the target nucleic acid or one member of the pair of FRET hybridization probes is already by immobilized on a solid support. The solid support itself, for example can be a hybridization membrane, a magnetic glass bead, a micro-array for immobilizing nucleic acids or any other material known in the art.

In another preferred embodiment, the nucleic acid to become detected in said sample is amplified by a nucleic acid amplification reaction, in particular by a polymerase chain reaction. More precisely, a part of said nucleic acid present in the sample is being subjected to a nucleic acid amplification reaction prior or during the hybridization procedure, for example, a polymerase chain reaction (PCR). As a prerequisite, the target nucleic acid comprises a sequence substantially complementary or homologous to the sequence of the used hybridization probes. In other words, the used hybridization probes need to hybridize specifically to the part of the target nucleic acid, which is being amplified.

In a specific embodiment, fluorescence emission of either the FRET donor entity or emission of the FRET acceptor entity is monitored in real time during the amplification reaction itself. Thus, it is also within the scope of the present invention, if a pair of FRET hybridization probes according to the invention is being used for monitoring progress of the amplification of a target nucleic during thermocycling. As it is known in the art, real time monitoring allows the generation of kinetic data and facilitates quantitative analysis. Thus, the present invention is also directed to a method of monitoring the amplification of a target nucleic acid by means of monitoring either the increase in fluorescence emission of the FRET acceptor entity or monitoring the decrease in fluorescence emission of the FRET donor entity during the amplification reaction itself.

In another aspect, the new invention provides a method for the determination of the melting profile of a hybrid consisting of a target nucleic acid and a pair of FRET hybridization probes disclosed above, characterized in that the fluorescence emission is determined as a function of temperature. In this aspect, the present invention is directed to the usage of the FRET hybridization probes disclosed above for melting curve analysis, wherein monitoring of the dissociation of a complex between a target nucleic acid and a hybridization probe allows for the detection of small sequence variants such as single nucleotide polymorphisms.

More precisely, the invention is directed to a method for the determination of the melting profile of a hybrid consisting of a target nucleic acid and a pair of FRET hybridization probes according to the invention, characterized in that first, a ternary hybrid complex between the target nucleic acid and the two hybridization probes is formed. Subsequently, the temperature is increased and the thermal dissociation of the ternary complex is determined by means of monitoring fluorescence in real time. In other words, the new invention is also directed to a method for the determination of the melting profile of a hybrid consisting of a target nucleic acid and a pair of FRET hybridization probes as disclosed above, characterized in that the fluorescence emission is determined as a function of temperature.

In a further aspect, the invention also provides a kit comprising a pair of hybridization probes according to invention as disclosed above. Preferably, at least one other component selected from a group consisting of nucleic acid amplification primers, template dependent nucleic acid polymerase, deoxynucleoside triphosphates and a buffer for template dependent nucleic acid amplification reaction.

In a last aspect, the present invention is directed to a kit comprising a pair of hybridization probes according to the invention. Such a kit may comprise a pair of FRET hybridization probes according to the invention as disclosed above. In addition, it may also contain oligonucleotides capable of acting as a primer pair for a nucleic acid amplification reaction.

In addition, a kit according to the present invention may contain at least one additional component such as a nucleic acid polymerase, deoxynucleoside triphosphates or respective analogues and an appropriate buffer which may be used for a template dependent nucleic acid amplification reaction such as PCR. Furthermore, the kit may also comprise software tools such as compact discs carrying computer programs for quantitative analysis of relative or absolute nucleic acid quantification experiments.

In general, the nucleotide residues carrying the spacer linked to the fluorescent moiety in principle may be either internal, 5' terminal or 3' terminal residues, as long as upon hybridization of the pair of oligonucleotides to the target nucleic acid, fluorescence resonance energy transfer can take place to an extent, wherein both FRET entities are brought into reasonable spatial vicinity such that subsequent to excitation of the FRET donor, fluorescence emission from the FRET acceptor can be monitored.

In this regard, the oligonucleotides acting as FRET hybridization probes may be labeled with the required fluorescent entity at any position by methods known in the art. For example, the oligonucleotides may be labeled internally at the nucleoside base or the phosphate moiety.

Preferably, however, and independently from the presence or absence of the spacer moiety, one oligonucleotide carrying the first FRET entity is labeled at the 3-hydroxy group of its 3' terminal residue and the second oligonucleotide carrying the second FRET entity is labeled at the 5' phosphate group of its 5' terminal residue such that when both oligonucleotides are hybridized to the target nucleic acid, the fluorescent labels of both FRET entities are brought in reasonable vicinity to each other due to the fact that said terminal residues are base pairing to adjacent residues in the target nucleic acid or at least to residues which are only separated by one, two or at maximum 10 further residues.

If one oligonucleotide is labeled at the 5' end and the second oligonucleotide is labeled at the 3' end, it may be chosen arbitrarily, which oligonucleotide carries the FRET donor moiety and which oligonucleotide carries the FRET acceptor moiety. Yet, as already discussed above, for the purpose of an improved fluorescent signaling, however, it has been proven to be advantageous, if the oligonucleotide carrying the spacer entity is labeled with the FRET donor moiety.

Usually, the 5' fluorescent label may be introduced at the end of the oligonucleotide synthesis using an appropriate Phosphor Amidate carrying a fluorescent compound. Alternatively, after oligonucleotide synthesis, an oligonucleotide carrying a reactive amino group may be labeled with a fluorescent compound activated as an NHS ester. For the 3' labeling of oligonucleotides, commercially available controlled pore glass particles are used as a solid support for the start of a chemical oligonucleotide synthesis, which comprise a tri-functional spacer entity with a fluorescent compound.

In general, the design if hybridization probes according to the invention is applicable to any combination of fluorescent compounds, between which fluorescent energy transfer may take place. Illustratory examples which are not at all limiting the invention are Fluorescein/Cy5 (Amersham), Fluorescein/LC-Red-640 (Roche Applied Science), Fluorescein/LC-Red-705 (Roche Applied Science), and Fluorescein/JA286 (EP 747 447).

It is also within the scope of the invention, if the FRET acceptor entity is a quencher moiety different from a fluorescent compound and consequently, decrease in fluorescence from the FRET donor moiety is monitored. Examples for quencher compounds which may be used in this regard are Dabcyl (Kreuzer, K. A., et al., Clin Chem 47 (2001) 486-90.) or so called Black Hole Quenchers (WO 01/86001)

In most cases, FRET hybridization probes are typical single stranded DNA molecules. Nevertheless, any kind of modification is possible. For example, the single stranded DNA may contain non-natural bases such as 7-deaza-purine, diamino-purine or C-nucleotides. The single stranded DNA may also have a modified sugar-phosphate backbone such as 2-O-Mehtyl, Phosphothioate, or anything similar.

In a still further aspect, the present invention is directed to a new strategy for efficient synthesis of oligonucleotides according to the invention with regard to the embodiment characterized in that the spacer entity is represented by a chain of additional oligonucleotide residues not hybridizing to the respective target DNA.

In case of applying the claimed invention to a multitude of different target sequences, there is a requirement to provide a multitude of different FRET hybridization probes, only having in common the same 5' or 3' end. In case of a common 3' end, solid phase oligonucleotide synthesis using a fluorescently labeled controlled pore glass particle (CPG) may be performed to provide an intermediate CPG-(N)n carrying a terminal hydroxy group which is protected by a protective group known in the art.

Such a reagent can subsequently be used as a solid phase starting material for solid phase synthesis of a multitude of desired different oligonucleotides having a common 3' end. Preferably, such a labeling reagent is composed of only one type of nucleotide residues, namely homo-A, homo-T or homo-C, having a size of n=1-10. Most preferably such a labeling reagent is composed of homo-T. The most preferred size is between 3 and 5.

Thus, the present invention is also directed to a method for chemical solid phase synthesis of multiple oligonucleotides comprising
a) Preparation of a dye labeled CPG- (N)n, characterized in that N are arbitrarily chosen nucleotide residues different from G and n=1-10
b) Solid phase synthesis of a first oligonucleotide having a first sequence using the CPG prepared in step a)
c) Solid phase synthesis of at least a second oligonucleotide having a second sequence using the CPG prepared in step a)

The method has been proven to be particular advantageous, in case a Fluorescein labeled CPG is used in order to prepare a labeling reagent for respective FRET donor probes.

The following examples, references, sequence listing and figures are provided to aid the understanding of the present invention, the true scope of which is set forth in the appended claims. It is understood that modifications can be made in the procedures set forth without departing from the spirit of the invention.

### Example 1

### Preparation of PCR primers and probes

Primers were synthesized on a 1 µmol scale on ABI 394 synthesizer using commercially available standard phosphoramidites ( DMTr ibu G, DMTr bzA; DMTr bz C and DMTr T) and the corresponding CPG support. The chemicals for standard synthesis were obtained from GlenResearch. Removal of the oligonucleotides from the solid support and deprotection was carried out with 33 % NH₃ for 8h at 55 °C. Synthesis was performed in the trityl on modus. Purification was done on a RP 18 Oligo R3 4.6 x 50 mm column from Perseptive Biosystems) Buffer A: 0.1M Triethylammonium acetate in water pH 7.0 /MeCN 95:5 buffer B: MeCN. gradient 3 min 20 % B; 12 min 12- 40 % B flow rate 1 ml/min detection 260 nm. Subsequently, the concentrated oligonucleotide solution was treated for 5 min with 80 % Acetic acid at room temperature in order to remove the 5' DMTr protecting group. Afterwards, oligonucleotides were desalted with a RP 18 column and lyophilyzed in a Speed Vac.

5' labeled Oligonucleotide (JA 286/ LC Red 640) synthesis was performed in the 1 µmol range. Commercially available standard phosphoramidites ( DMTr ibu G, DMTr bzA; DMTr bz C and DMTr T) and chemicals for standard synthesis were obtained from Glen Research. The 5' amino group was introduced by using commercially available 5' amino modifier (Glen Research (cat no. 10-1916-90) As solid support 3' phosphate CPG (GlenResearch 20-2900-01) was used. Removal of the oligonucleotides from the solid support and deprotection was carried out with 33 % NH₃ for 8h at 55 °C. The solution was evaporated under vacuum. The remainder was dissolved in 600 µl double destilled water and transferred in a microcentrifuge tube 60 µl of sodium acetate buffer (3M, ph 8.5 were added). Upon addition of 1.8 ml ice cold ethanol the mixture was stored at -15 °C for 3 h. The solution was centrifuged at 10000 x g for 15 min. The supernatant was decanted. The pellet is washed with 200 µl ice cold ethanol. After centrifugation the supernatent was decanted. The pellet was dissolved in 400 µl sodium borate buffer (0.1M pH 8.5) and was labeled according standard procedures.

NHS-activated LC-Red-640 and JA286 were used. NHS-LC-Red-640 is obtainable from Roche applied Science (Cat. No: 2 015 161). NHS activated JA286 was synthesized according to EP 0747 447, example 1. NHS activated Cy5 was obtained from Amersham (Cat. No. DEITER?), whereas LCRed 705 (Roche Applied Science Cat. No. 2 157 594) was incorporated during oligonucleotide synthesis in form of a Phospohoramidate.

A solution of 1 mg of the dye NHS ester in DMF was added and reacted for 15 h The labeled oligonucleotide was purified by reversed phase using a Oligo R3 4.6 x 50 mm column) Chromatography: buffer A: 0.1M Triethylammoniamacetat in water pH 7.0 buffer B: 0.1 M triethylammonium acetate in water/MeCN 1 : 1. gradient 2 min 0 % B in 45 min to 100 %B ( the gradient was stopped when a product starts to eluate at 20 -25 % B the nonlabeled oligonucloetide eluates; at 60 -65 % B the desired labeled oligonucleotide eluates at 100 % B the dye eluates; flow rate was 1 ml/min detection at 260 nm. The fractions from the labeled oligonucleotide peaks were collected and the solvent was removed by using a vacuum centrifuge. The remainder was dissolved in double distilled water and then evaporated again with vacuum centrifuge, This procedure was repeated three times. The pellet was dissolved in water and lyophilized.

3' Fluorescein labeled oligonucleotides were synthesized and purified according to the pack insert of the commercially available LightCycler Fluorescein CPG (Roche Applied Science cat no. 3138178). This specific CPG confers a C12 linker to the nucleotide to become synthesized (EP 1 186 613).

### Example 2

### Quantitative real time PCR of Factor V DNA using FRET hybridization probes labeled with Fluorescein/ JA286 with 1 spacer according to the invention

For amplification of a Factor V DNA fragment, 20µl Real Time PCR reaction mixtures were set up as follows:

| | |
|---|---|
| 10⁶ | copies of a plasmid containing the Factor V gene (Gene Bank Accession No: M_014335 ) |
| 3 mM | MgCl₂ |
| 500 nM each | primers according to SEQ. ID. NO: 1 and 2 |
| 200 nM each | FRET hybridization probes according to SEQ.ID. NO: 3 and 4, 3 and 6, 3 and 8, 4 and 5, 4 and 7, respectively. |

PCR components of LightCycler DNA Master Hyb Probes Kit (Roche Applied Science, Cat. No. 2158825)

Primers and probes were used as follows:

SEQ ID NO:1
Forward primer:
5' GAG AGA CAT CGC CTC TGG GCT A

SEQ ID NO:2
Reverse Primer
5' TGT TAT CAC ACT GGT GCT AA

SEQ ID NO:3
FRET donor probe
5' AAT ACC TGT ATT CCT CGC CTG TC-Fluorescein

SEQ ID NO:4
FRET acceptor probe
5' JA286-AGG GAT CTG CTC TTA CAG ATT AGA AGT AGT CCT ATT

SEQ ID NO:5
FRET donor probe
5‘ AAT ACC TGT ATT CCT CGC CTG TCA AAA A-Fluorescein

SEQ ID NO:6
FRET acceptor probe
5' JA286-TTT  TTA  GGG ATC TGC TCT TAC AGA TTA GAA GTA GTC CTA TT

SEQ ID NO:7
FRET donor probe
5‘ AAT ACC TGT ATT CCT CGC CTG TCA AA-Fluorescein

SEQ ID NO:8
FRET acceptor probe
5' JA286-TTT AGG GAT CTG CTC TTA CAG ATT AGA AGT AGT CCT ATT

The probes according to SEQ.ID.NO: 3, 5 and 7 were 3' terminally labeled with Fluorescein according to example 1. The probes according to SEQ. ID. NO: 4, 6 and 8 were 5' terminally labeled with JA286 as a FRET acceptor according to example 1.

The FRET donor probe according to SEQ. Id. NO: 3 was used in combination with a FRET acceptor probe according to SEQ. ID. NO: 4 as a negative control. In addition, donor probes according to SEQ.ID.NO: 5 or SEQ. ID. NO:7 comprising 5 or 3 A nucleotide residue spacers according to the invention were combined with the FRET acceptor probe according to SEQ. ID. NO: 4. Alternatively, The FRET donor probe according to SEQ. Id. NO: 3 was used in combination with FRET acceptor probe according to SEQ. ID. NO: 6 or 8 comprising 5 or 3 T nucleotide residue spacers according to the invention.

Amplification was performed in a LightCycler instrument (Roche Applied Science) according to the following thermocycling protocol:

**Table 1:**

| | T[°C] | t[sec] | Ramp-rate[°C/sec] | Acquisition | Cycles |
|---|---|---|---|---|---|
| Denaturation | 95 | 30 | 20.0 | none | 1 |
| Amplification | 95 | 0 | 20.0 | none | |
| | 55 | 10 | 20.0 | single | 45 |
| | 72 | 10 | 20.0 | none | |

Real time monitoring was performed using the 2^{nd} derivative threshold method over 45 cycles by measuring the fluorescence signals in a detection channel specific for JA286 emission (at 710 nm) and using arithmetic background correction for normalization of initial fluorescence background intensities.

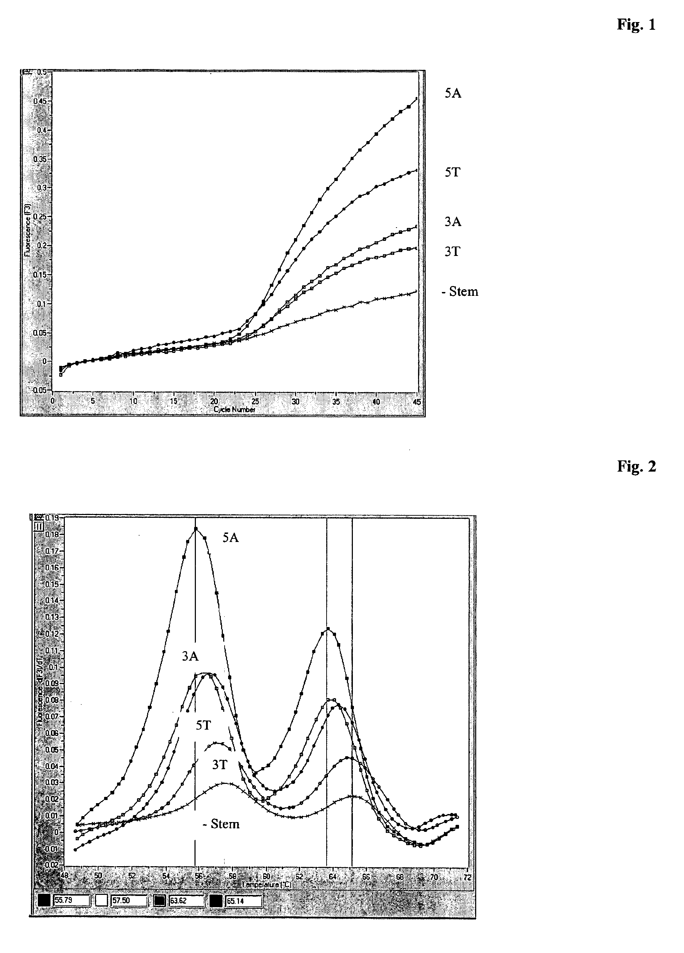

The result is shown in fig. 1. As can be seen in the figure, an improved amplification signal was obtained with a spacer consisting of either 3 or 5 A residues in case of the Fluorescein labeled FRET donor probe, or, alternatively 3 or 5 T residues in case of the JA286 labeled FRET acceptor probe. The effect was observed independently from whether the stem was either part of the FRET donor probe or the FRET acceptor probe, but stronger fluorescent signals could be obtained in case the FRET donor probe was designed according to the invention.

The observed result is especially surprising, because introduction of only one stem into a pair of FRET hybridization probes was supposed to be disadvantageous in view of the increase in distance between the FRET donor moiety and the FRET acceptor moiety.

### Example 3

### Melting Curve Analysis of Factor V DNA using hybridization probes according to the invention, labeled with Fluorescein/ JA286 having 1 spacer according to the invention

Subsequent to the reaction disclosed in example 2, the samples were subjected to a melting curve analysis according to the instructions of the LightCycler manual (Roche Applied Sciences) using the following temperature transition protocol:

**Table 2:**

| | T[°C] | t[sec] | Ramp-rate[°C/sec] | Acquisition | Cycles |
|---|---|---|---|---|---|
| Melting curve | 95 | 0 | 20.0 | none | |
| | 45 | 60 | 20.0 | continuous | 1 |
| | 75 | 10 | 0.1 | none | |
| Cooling | 40 | 30 | 20.0 | none | 1 |

Fluorescence monitoring was performed by measuring the absolute signal values obtained in the JA286 channel at 710 nm and subsequent calculation of the first derivative.

The result is shown in fig. 2. As can be seen in the figure, usage of FRET hybridization probes characterized in that either the FRET donor probe or, alternatively, the FRET acceptor probe had a 3 or 5 nucleotide residue spacer moiety according to the invention, in both cases also resulted in increased melting peaks as compared to a pair of FRET hybridization probes without any spacer.

### Example 4

### Quantitative real time PCR of G6PDH DNA using FRET hybridization probes according to the invention, but different FRET acceptor dyes

The experiment was performed as disclosed in example 2 with the modifications that G6PDH as a different target DNA sequence was detected and the utility of different FRET acceptor dyes was compared and a concentration of 4mM MgCl2 was used.

Thus, primers and probes were as follows:

SEQ ID NO:9
Forward primer
5' GGG TGC ATC GGG TGA CCT G

SEQ ID NO:10
Reverse primer
5' AGC CAC TGT GAG GCG GGA

SEQ ID NO:11
FRET donor probe
5' GGT GTT TTC GGG CAG AAG GCC ATC C- Fluorescein

SEQ ID NO:12
FRET acceptor probes:
5' JA286-AAC AGC CAC CAG ATG GTG GGG TAG ATC TT
5' LCRed640- AAC AGC CAC CAG ATG GTG GGG TAG ATC TT
5'-Cy5- AAC AGC CAC CAG ATG GTG GGG TAG ATC TT
5'-LCRed705- AAC AGC CAC CAG ATG GTG GGG TAG ATC TT

SEQ ID NO:13
FRET donor probe:
5'-GGT GTT TTC GGG CAG AAG GCC ATC CAA AAA

The probes according to SEQ.ID.NO: 11 and 13 were 3' terminally labeled with Fluorescein according to example 1. The probes according to SEQ. ID. NO: 12 was 5' terminally labeled with JA286, LCRed640, Cy5 or LCRed705 as a FRET acceptor according to example 1. The FRET donor probes according to SEQ. Id. NO: 11 and 13 used in combination with all four FRET acceptor probes according to SEQ. ID. NO:12 each labeled with a different acceptor dye.

Amplification was performed in a LightCycler instrument (Roche Applied Science) according to the following thermocycling protocol:

**Table 3:**

| | T[°C] | t[sec] | Ramp-rate[°C/sec] | Acquisition | Cycles |
|---|---|---|---|---|---|
| Denaturation | 95 | 60 | 20.0 | none | 1 |
| Amplification | 95 | 0 | 20.0 | none | |
| | 55 | 15 | 20.0 | single | 45 |
| | 72 | 15 | 20.0 | none | |
| Cooling | 40 | 30 | 20.0 | none | 1 |

The result is shown in fig. 3a-d. As can be seen in the figure, an improved amplification signal was obtained for all FRET pairs, wherein the FRET donor probe comprised a stem according to the invention as compared to a FRET pair with the same fluorescent dyes not comprising such a stem.

It can be concluded that the inventive improvement is applicable independently from the target DNA to be detected and, even more important, independently from the FRET acceptor dye which is actually used.

### Example 5

### Quantitative Real time PCR of Factor V DNA using FRET hybridization probes labeled with different spacers according to the invention

The experiment was performed as disclosed in example 2 with the modification that FRET donor probes with different nucleotide residue spacers according to the invention were tested.

Primers and probes were used as follows:

SEQ ID NO:1
Forward primer:
5' GAG AGA CAT CGC CTC TGG GCT A

SEQ ID NO:2
Reverse Primer
5‘ TGT TAT CAC ACT GGT GCT AA

SEQ ID NO:3
FRET donor probe
5' AAT ACC TGT ATT CCT CGC CTG TC-Fluorescein

SEQ ID NO:4
FRET acceptor probe
5' JA286-AGG GAT CTG CTC TTA CAG ATT AGA AGT AGT CCT ATT
5' LCRed705-AGG GAT CTG CTC TTA CAG ATT AGA AGT AGT CCT ATT

SEQ ID NO:5
FRET donor probe
5' AAT ACC TGT ATT CCT CGC CTG TCA AAA A-Fluorescein

SEQ ID NO:14
FRET donor probe
5' AAT ACC TGT ATT CCT CGC CTG TCT TTT T-Fluorescein

The probes according to SEQ.ID.NO: 3, 5 (containing a 5 A spacer) and 14 (containing a 5 T spacer) were 3' terminally labeled with Fluorescein according to example 1. The probes according to SEQ. ID. NO: 4 were 5' terminally labeled with either JA286 or LCRed705 as a FRET acceptor according to example 1. The three different FRET donor probes each were used in combination with the two different FRET acceptor probe according to SEQ. ID. NO: 4.

The result is shown in fig. 5a and b. As can be seen in the figure, usage of different FRET donor hybridization probes with different nucleotide stem residues as spacers according to the invention always resulted in an improved fluorescent signaling as compared to the usage of a FRET donor hybridization probe without any spacer moiety. The observed effect was independent from the detected target sequence.

### List of References

Bernard, P. S., et al., Anal Biochem 255 (1998) 101-7.
Kreuzer, K. A., et al., Clin Chem 47 (2001) 486-90.
Matthews, J. A. and Kricka, L. J., Anal Biochem 169 (1988) 1-25.
Seidel, C. A. M., et al., J Phys Chem 100 (1996) 5541-53
EP 0747 447
EP 1 046 717
EP 1 186 613
EP 747 447
US 5,118,801
US 5,538,848
WO 01/36668
WO 01/73118
WO 01/86001
WO 97/46707
WO 97/46712
WO 97/46714

## Claims

1. A pair of FRET hybridization probes hybridizing adjacently to a target nucleic acid sequence, a first member of said pair of hybridization probes comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity, said spacer entity comprising a connecting chain of at least 15 atoms,
wherein 2 atoms of said connecting chain of at least 15 atoms are carrying negatively charged substituents.

2. A pair of FRET hybridization probes according to claim 1, wherein said spacer entity is 1-10, preferably 2-7 and most preferred 3-5 A, T, or C nucleotide residues, which are not capable of hybridizing to the target DNA.

3. A pair of FRET hybridization probes hybridizing adjacently to a target nucleic acid sequence, the first member of said pair of FRET hybridization probes comprising
- a nucleotide sequence entity which is substantially complementary to the sequence of the target nucleic acid
- a fluorescent entity, said entity being the FRET donor entity
- a first spacer entity connecting said nucleotide sequence entity and said first fluorescent entity,
the second member of said pair of FRET hybridization probes comprising
- a nucleotide sequence entity which is substantially complementary to the sequence of the target nucleic acid
- a second fluorescent entity, said entity being the FRET acceptor entity
- a second spacer entity connecting said nucleotide sequence entity and said fluorescent entity, said spacer entity being different from the spacer entity of said first member of said pair of FRET hybridization probes,
wherein the length of said first spacer entity the length of said second spacer entity differ in size at least by a connecting chain of 15 atoms.

4. A pair of FRET hybridization probes hybridizing adjacently to a target nucleic acid sequence, the first member of said pair of FRET hybridization probes comprising
- a nucleotide sequence entity which is substantially complementary to the sequence of the target nucleic acid
- a fluorescent entity, said entity being the FRET donor entity
- a spacer entity connecting said nucleotide sequence entity and said fluorescent entity, said spacer entity comprising a number of n1=0-15 nucleotide residues which are not capable of hybridizing to the target DNA
the second member of said pair of FRET hybridization probes comprising
- a nucleotide sequence entity which is substantially complementary to the sequence of the target nucleic acid
- a fluorescent entity, said entity being the FRET acceptor entity
- a spacer entity connecting said nucleotide sequence entity and said fluorescent entity, said spacer entity comprising a number of n2=0-15 nucleotide residues which are not capable not hybridizing to the target DNA,
wherein the value of n1 differs from the value of n2 by a natural number between 1 and 10, preferably by a natural number between 2-7 and most preferably by a natural number between 3-5.

5. A set of at least 3 oligonucleotides, **characterized in that** a first oligonucleotide and a second oligonucleotide are capable of acting as a pair of amplification primers for a template dependent nucleic acid amplification reaction, further **characterized in that** said first oligonucleotide and said third oligonucleotide are each labeled with one corresponding member of a FRET pair consisting of a FRET donor entity and a FRET acceptor entity,
either said first said oligonucleotide or said third oligonucleotide comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity,
wherein said spacer entity comprises a connecting chain of at least 15 atoms, preferably consists of a chain of 1-10 and most preferably consists of a chain of 3-5 nucleotide residues.

6. A composition comprising a nucleic acid sample and a pair of hybridization probes according to claims 1-5

7. A kit comprising a pair of hybridization probes according to claims 1-5 and at least one other component selected from a group consisting of nucleic acid amplification primers, template dependent nucleic acid polymerase, deoxynucleoside triphosphates and a buffer for template dependent nucleic acid amplification reaction.

8. Method for qualitative or quantitative detection of a nucleic acid sequence in a biological sample, wherein the nucleic acid present in said sample is hybridized with a pair of FRET hybridization probes according to claim 1-5.

9. Method according to claim 8, wherein a part of said nucleic acid present in said sample, which comprises a target nucleic acid sequence substantially complementary to the sequences of said pair of FRET hybridization probes is amplified by a nucleic acid amplification reaction, in particular by a polymerase chain reaction.

10. Method according to claim 9, wherein fluorescence emission of either the FRET donor entity or emission of the FRET acceptor entity is monitored in real time.

11. Method for the determination of the melting profile of a hybrid consisting of a target nucleic acid and a pair of FRET hybridization probes according to claims 1-5, **characterized in that** the fluorescence emission is determined as a function of temperature.

12. Method for chemical solid phase synthesis of multiple oligonucleotides comprising
a) Preparation of a dye labeled CPG- (N)n,
**characterized in that** N are arbitrarily chosen nucleotide residues different from G and n=1-10
b) Solid phase synthesis of a first oligonucleotide having a first sequence using the CPG prepared in step a)
c) Solid phase synthesis of at least a second oligonucleotide having a second sequence using the CPG prepared in step a)


SEQUENCE LISTING

<110> Roche Diagnostics GmbH
      F. HOFFMANN-LA ROCHE AG

<120> Improved FRET Process

<130> 21428 EP

<140>
<141>

<160> 14

<170> PatentIn Ver. 2.1

<210> 1
<211> 22
<212> DNA
<213> Artificial Sequence

<220>
<223> Description of Artificial Sequence:primer

<400> 1
gagagacatc gcctctgggc ta                                          22

<210> 2
<211> 20
<212> DNA
<213> Artificial Sequence

<220>
<223> Description of Artificial Sequence:primer

<400> 2
tgttatcaca ctggtgctaa                                             20

<210> 3
<211> 23
<212> DNA
<213> Artificial Sequence

<220>
<223> Description of Artificial Sequence:primer

<400> 3
aatacctgta ttcctcgcct gtc                                         23

<210> 4
<211> 36
<212> DNA
<213> Artificial Sequence

<220>
<223> Description of Artificial Sequence:primer

<400> 4
agggatctgc tcttacagat tagaagtagt cctatt                                36

<210> 5
<211> 28
<212> DNA
<213> Artificial Sequence

<220>
<223> Description of Artificial Sequence:primer

<400> 5
aatacctgta ttcctcgcct gtcaaaaa                                         28

<210> 6
<211> 41
<212> DNA
<213> Artificial Sequence

<220>
<223> Description of Artificial Sequence:primer

<400> 6
tttttaggga tctgctctta cagattagaa gtagtcctat t                          41

<210> 7
<211> 26
<212> DNA
<213> Artificial Sequence

<220>
<223> Description of Artificial Sequence:primer

<400> 7
aatacctgta ttcctcgcct gtcaaa                                           26

<210> 8
<211> 39
<212> DNA
<213> Artificial Sequence

<220>
<223> Description of Artificial Sequence:primer

<400> 8
tttagggatc tgctcttaca gattagaagt agtcctatt                             39

<210> 9
<211> 19
<212> DNA

<213> Artificial Sequence

<220>
<223> Description of Artificial Sequence:primer

<400> 9
gggtgcatcg ggtgacctg                                                    19


<210> 10
<211> 18
<212> DNA
<213> Artificial Sequence

<220>
<223> Description of Artificial Sequence:primer

<400> 10
agccactgtg aggcggga                                                     18


<210> 11
<211> 25
<212> DNA
<213> Artificial Sequence

<220>
<223> Description of Artificial Sequence:primer

<400> 11
ggtgttttcg ggcagaaggc catcc                                             25


<210> 12
<211> 29
<212> DNA
<213> Artificial Sequence

<220>
<223> Description of Artificial Sequence:primer

<400> 12
aacagccacc agatggtggg gtagatctt                                         29


<210> 13
<211> 30
<212> DNA
<213> Artificial Sequence

<220>
<223> Description of Artificial Sequence:primer

<400> 13
ggtgttttcg ggcagaaggc catccaaaaa                                        30

<210> 14
<211> 28
<212> DNA
<213> Artificial Sequence

<220>
<223> Description of Artificial Sequence:primer

<400> 14
aatacctgta ttcctcgcct gtcttttt                                    28